# EUROPEAN PATENT SPECIFICATION

(11) **EP 1 824 447 B1**
(45) Date of publication and mention of the grant of the patent: **09.06.2010**
(21) Application number: 05850384.8
(22) Date of filing: 22.11.2005
(51) Int. Cl.: A61Q 19/08, A61K 8/42, A61K 8/25, A61K 8/87

(54) **COSMETIC COMPOSITION COMPRISING A TENSIONING AGENT AND A HYDROXYALKYL UREA**
KOSMETISCHE ZUSAMMENSETZUNG MIT EINEM STRAFFUNGSMITTEL UND HYDROXYALKYL-HARNSTOFF
COMPOSITION COSMÉTIQUE COMPRENANT UN AGENT TENSEUR ET UNE HYDROXYALKYLURÉE

(30) Priority: 26.11.2004 FR 0452768; 09.12.2004 US 634283 P
(43) Date of publication of application: 29.08.2007
(73) Proprietor: L'Oréal, 75008 Paris (FR)
(72) Inventor: CASSIN, Guillaume, F-91140 Villebon sur Yvette (FR)
(74) Representative: Kromer, Christophe
(86) International application number: PCT/EP2005/014176
(87) International publication number: WO 2006/056491

(56) References cited:
- EP-A- 0 434 179
- EP-A- 1 535 607
- WO-A-2006/018198
- DE-A1- 2 703 185
- FR-A- 2 823 113

## Description

The present invention relates to a cosmetic composition, in particular an anti-wrinkle composition, comprising, in a physiologically acceptable medium suitable for topical application to facial skin: (a) at least one tensioning agent chosen from:
- colloidal particles of silica or of silica-alumina composite
- synthetic polymer chosen from interpenetrating polymer networks comprising a polyurethane polymer and a polyacrylic polymer,
and (b) at least one hydroxyalkyl urea of given formula.

It also relates to the use of this hydroxyalkyl urea for improving the remamence of the tensioning effect afforded by a tensioning agent, and also to a process for treating wrinkled skin by applying to said skin a composition comprising this hydroxyalkyl urea and a tensioning agent.

The general field of the invention is therefore that of ageing of the skin.

In the course of ageing of the skin, various signs appear, reflected in particular by a change in the structure and functions of the skin. One of these main signs is the appearance of fine lines, and deep wrinkles, the size and the number of which increase with age. The microrelief of the skin becomes less even and exhibits an anisotropic characteristic.

It is common practice to treat these signs of ageing with cosmetic compositions containing active agents capable of combating ageing, such as α-hydroxy acids, β-hydroxy acids, ascorbic acid and retinoids. These active agents act in particular on wrinkles by eliminating the dead cells of the skin and accelerating the process of cell renewal and/or by increasing the synthesis of extracellular matrix components (glycosaminoglycans, collagen, elastin) or preventing degradation thereof. However, these active agents have the drawback of being effective in treating wrinkles only after they have been applied for a certain amount of time, i.e. an amount of time that may range from a few days to several weeks.

Now, current needs are increasingly tending towards the production of compositions for obtaining an immediate effect, leading rapidly to smoothing-out of the wrinkles and/or fine lines and to the disappearance, even temporary, of fatigue marks. Such compositions are compositions comprising tensioning agents. It is specified that the term "tensioning agent" is intended to mean compounds capable of having a tensioning effect, i.e. compounds that can make the skin taut and bring about a reduction in or even the immediate disappearance of wrinkles, fine lines and fatigue marks. These tensioning agents may in particular be polymers of natural or synthetic origin in an aqueous dispersion, capable of forming a film that causes shrinkage of the stratum corneum, the superficial or horny layer of the epidermis. The cosmetic or dermatological use of such polymer systems for attenuating the effects of ageing of the skin is described in Patent Application WO 98/29091. Other tensioning agents consist of dispersions of inorganic colloidal particles, in particular of silica, as described in Patent Applications FR-A-2 823 113, FR-2 843 024 and FR-2 659 551 or in US Patents 3,819,825 and 4,777,041, for example. Other additional tensioning agents are mixed silicates such as those described in Application FR-2 816 315.

However, these tensioning agents sometimes give a sensation of discomfort to certain users, in particular those who have fragile skin. In addition, the tensioning effect that they afford does not last very long, since the film formed on the skin has a tendency to crack as a result of the facial expressions. The reason for this is that these tensioning agents form a relatively rigid and inflexible film on the skin.
The applicant has noted, surprisingly, that the use of specific hydroxyalkyl ureas in combination with a particular tensioning agent in a cosmetic composition makes it possible to obtain films that have a satisfactory and long-lasting tensioning effect, said films being flexible and deformable from a mechanical point of view.

Thus, according to a first subject, the invention relates to a cosmetic composition comprising, in a physiologically acceptable medium suitable for topical application to facial skin:
(a) at least one tensioning agent chosen from: plant proteins and hydrolysates thereof, mixed silicates, colloidal particles of inorganic filler and synthetic polymers, and
(b) at least one compound chosen from:
   - hydroxyalkyl ureas corresponding to general formula (I) : 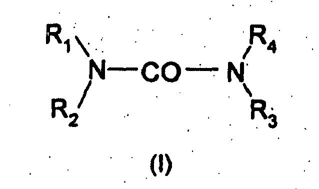 in which R₁, R₂, R₃ and R₄ each independently represent a hydrogen atom, a C₁-C₄ alkyl group or a C₂-C₆ hydroxyalkyl group that may contain from 1 to 5 hydroxyl groups, at least one of the radicals R₁-R₄ representing a hydroxyalkyl group, and
   - the salts, solvates thereof.

Among the alkyl groups, mention may be made of methyl, ethyl, n-propyl, isopropyl, n-butyl, isobutyl and tert-butyl groups.

The preferred compounds of formula (I) are those that contain just one hydroxyalkyl group, i.e. those for which R₁ is a hydroxyalkyl group and R₂, R₃ and R₄ represent, independently of one another, a hydrogen atom or a C₁-C₄ alkyl group. The compounds of formula (I) for which R₁ is a hydroxyalkyl group and R₂, R₃ and R₄ each represent a hydrogen atom are more particularly preferred.

Among the hydroxyalkyl groups, those containing a single hydroxyl group, and in particular the hydroxyethyl, hydroxypropyl, hydroxybutyl, hydroxypentyl and hydroxyhexyl groups, are preferred.

As preferred compounds of formula (I), mention may be made of N-(2-hydroxyethyl) urea; N-(2-hydroxypropyl) urea; N-(3-hydroxypropyl) urea; N-(2,3-dihydroxypropyl) urea; N-(2,3,4,5,6-pentahydroxyhexyl) urea; N-methyl-N-(1,3,4,5,6-pentahydroxy-2-hexyl) urea; N-methyl-N'-(1-hydroxy-2-methyl-2-propyl) urea; N-(1-hydroxy-2-methyl-2-propyl) urea; N-(1,3-dihydroxy-2-propyl) urea; N-(trishydroxymethylmethyl) urea; N-ethyl-N'-(2-hydroxyethyl) urea; N,N-bis-(2-hydroxyethyl) urea; N,N'-bis-(2-hydroxyethyl) urea; N,N-bis-(2-hydroxypropyl) urea; N,N'-bis-(2-hydroxypropyl) urea; N,N-bis-(2-hydroxyethyl)-N'-propyl urea; N,N-bis-(2-hydroxypropyl)-N'-(2-hydroxyethyl) urea; N-tert-butyl-N'-(2-hydroxyethyl)-N'-(2-hydroxypropyl) urea; N-(1,3-dihydroxy-2-propyl)-N'-(2-hydroxyethyl) urea; N,N-bis-(2-hydroxyethyl)-N',N'-dimethyl urea; N,N,N',N'-tetrakis-(2-hydroxyethyl) urea; N',N'-bis-(2-hydroxyethyl)-N',N'-bis-(2-hydroxypropyl) urea.

A compound that is particularly preferred for use in the present invention is N-(2-hydroxyethyl) urea, hereinafter referred to as "hydroxyethyl urea".

The hydroxyalkyl ureas of formula (I) can be prepared as described in application DE-27 03 185. Among these, hydroxyethyl urea is also commercially available, in the form of a mixture at 50% by weight in water, from the company National Starch under the trade name Hydrovance®.

Among the salts, mention may be made of salts of inorganic acids, such as sulphuric acid, hydrochloric acid, hydrobromic acid, hydriodic acid, phosphoric acid or boric acid. Mention may also be made of salts of organic acids, which may contain one or more carboxylic, sulphonic or phosphonic groups. They may be linear, branched or cyclic aliphatic acids or else aromatic acids. These acids may also contain one or more hetero atoms chosen from O and N, for example in the form of hydroxyl groups. Mention may in particular be made of propionic acid, acetic acid, terephthalic acid, citric acid and tartaric acid.

The term "solvate" is intended to mean a stoichiometric mixture of said compound of formula (I) with one or more molecules of water or of organic solvent, such a mixture being derived from the synthesis of the compound of formula (I).

The hydroxyalkyl urea advantageously represents from 0.01 to 20% by weight, and preferably from 1 to 10% by weight, relative to the total weight of the composition.

The other essential constituent of the composition according to the invention is a tensioning agent.

According to the invention, the term "tensioning agent" is intended to mean any agent that produces, at a concentration of 7% in water, a retraction of isolated stratum corneum, measured with an extensometer, of at least 0.9%, and preferably of more than 1.5%, at 30°C in a relative humidity of 40%.

The principle of the method consists in measuring the length of a test sample of stratum corneum isolated from human skin originating from a surgical operation, before and after treatment with the potential tensioning agent.

To do this, the test sample is placed between the two jaws of the device, one of which is fixed and the other mobile, in an atmosphere of 30°C and 40% relative humidity. A tensile stress is exerted on the test sample, and the curve of the force (in grams) as a function of the length (in millimetres) is recorded, the length zero corresponding to the contact between the two jaws of the device. The tangent to the curve is then plotted in its linear region. The intersection of this tangent with the x-axis corresponds to the apparent length L₀ of the test sample at zero force. The test sample is consequently slackened and 2 mg/cm² of the composition to be tested (7% solution of the tensioning agent under consideration) are then applied to the stratum corneum. After drying for 15 minutes, the above steps are again carried out in order to determine the length L₁ of the test sample after treatment. The percentage retraction is defined by: % retraction = 100 × (L₁-L₀)/L₀. In order to characterize a tensioning effect, this percentage must be negative, and the higher the absolute value of the percentage retraction, the greater the tensioning effect.

The tensioning agent is specifically chosen from:
- colloidal particles of silica or of silica-alumina composite
- synthetic polymer chosen from interpenetrating polymer networks comprising a polyurethane polymer and a polyacrylic polymer.

Those skilled in the art will be able to choose, from the chemical categories listed above, the materials corresponding to the tensioning test as described above.

These various categories of tensioning agents will now be described.

### c) Colloidal particles of inorganic filler

As another variant, use may be made, as tensioning agent according to the invention, of colloidal particles of inorganic fillers. The term "colloidal particles" is intended to mean particles in dispersion in an aqueous, aqueous-alcoholic or alcoholic medium, preferably an aqueous medium, having a number-average diameter of between 0.1 and 100 nm, preferably between 3 and 30 nm.

The colloidal particles according to the invention have no thickening property in water, alcohol, oil or any other solvents. At a concentration of greater than or equal to 15% by weight in water, the viscosity of the solution thus obtained is less than 0.05 Pa.s for a shear rate equal to 10 s⁻¹. The measurements are carried out at 25°C using a Haake RheoStress RS150 rheometer in the cone-plate configuration, the measurements of the measuring cone being: diameter: 60 mm and angle: 2°.

These particles are generally prepared according to a sol-gel process and therefore differ in particular from particles of fumed silica, which agglomerate in water so as to form aggregates larger in size.

Colloidal silica particles are in particular available in the form of an aqueous dispersion of colloidal silica from the company Catalysts & Chemicals under the trade names Cosmo S-40 and Cosmo S-50.

A specific example of colloidal particles may consist of colloidal particles of silica-alumina composite. The term "silica-alumina composite" is intended to mean silica particles in which the aluminium atoms have been partly substituted with silica atoms.

At a pH of 7, these colloidal particles of silica-alumina composite have a zeta potential of less than -20 mV, and preferably less than -25 mV. The measurements are carried out at 25°C using a Coulter Scientific Instrument Delsa 440SX device.

As silica-alumina composite colloidal particles that can be used in the compositions according to the invention, mention may, for example, be made of those sold by the company Grace under the names Ludox AM, Ludox AM X 6021, Ludox HSA and Ludox TMA.

### d) Synthetic polymers

The synthetic polymers that can be used as a tensioning agent can be chosen from: interpenetrating polymer networks (IPNs) comprising a polyurethane polymer and a polyacrylic polymer.

For the purposes of the present invention, the term "interpenetrating polymer network" is intended to mean a blend of two intermeshed polymers, obtained by simultaneous polymerization and/or crosslinking of two types of monomer, the blend obtained having a single glass transition temperature.

Examples of IPNs that are suitable for use in the present invention, and also the process for preparing them, are described in patents US-6,139,322 and US-6,465,001, for example.

According to a preferred embodiment, the IPN according to the invention comprises a polyurethane polymer and a polyacrylic polymer. Such IPNs are in particular those of the Hybridur series that are commercially available from the company Air Products.

An IPN that is particularly preferred is in the form of an aqueous dispersion of particles with a weight-average size of between 90 and 110 nm and a number-average size of approximately 80 nm. This IPN preferably has a glass transition temperature, Tg, that ranges from approximately -60°C to +100°C. An IPN of this type is in particular sold by the company Air Products under the trade name Hybridur X-01602. Another IPN that is suitable for use in the present invention has the reference Hybridur X18693-21.

The tensioning agent may be included in the composition according to the invention in an amount ranging from 0.01 to 20% by weight of active material, preferably from 1% to 10% by weight of active material, relative to the total weight of the composition. The term "active material" is intended to exclude the medium in which the tensioning agent is optionally solubilized or in dispersion in its commercial form, for example in the case of dispersions of colloidal particles.

The composition according to the invention comprises a physiologically acceptable medium, i.e. a medium compatible with facial skin. It is preferably a cosmetically acceptable medium, i.e., a medium with a pleasant colour, odour and feel, and which does not generate any unacceptable discomfort (stinging, tightness, redness), that may turn the consumer away from using this composition.

The composition according to the invention may be in any of the pharmaceutical forms conventionally used for topical application, and in particular in the form of dispersions of the aqueous lotion or gel type, of emulsions with a liquid or semiliquid consistency of the milk type, obtained by dispersion of a fatty phase in an aqueous phase (O/W) or vice versa (W/O), or of suspensions or emulsions with a soft, semi-solid or solid consistency of the cream or gel type, or else of multiple emulsions (W/O/W or O/W/O). These compositions are prepared according to the usual methods.

According to a preferred embodiment of the invention, the composition is in the form of an emulsion, and more particularly of an oil-in-water (O/W) emulsion.

This composition may also contain various adjuvants commonly used in the cosmetics field, such as emulsifiers; fillers; preserving agents; sequestering agents; fragrances; thickeners and/or gelling agents.

Of course, those skilled in the art will take care to select this or these optional additional compound(s) and/or the amount thereof in such a way that the advantageous properties of the composition according to the invention are not, or are not substantially, impaired by the envisaged addition.

The composition according to the invention may also contain anti-ageing active agents having an effect complementary to the polymers defined above, such as at least one compound chosen from agents for stimulating collagen and/or elastin synthesis or for preventing degradation thereof, anti-glycation agents, dermo-decontracting agents or muscle-relaxing agents, and mixtures thereof.

Finally, a subject of the present invention is a process for cosmetically treating wrinkled skin, comprising a step consisting in applying to said skin a composition containing a hydroxyalkyl urea as defined above and at least one tensioning agent as defined above.

The application is carried out according to the usual techniques, for example by application of creams, gels, sera or lotions to the skin intended to be treated, in particular the skin around the eyes. In the context of this process, the composition may, for example, be a care composition or a makeup composition.

According to another aspect, the invention also relates to a cosmetic assembly comprising:
i) a container delimiting at least one compartment, said container being closed by means of a closing member; and
ii) a composition as defined above and placed inside said compartment.

The container may be in any appropriate form. It may in particular be in the form of a bottle, a tube, a jar, a case, a box, a sachet or a carton.

The closing member may be in the form of a removable stopper, a lid, a cap, a tear-off strip or a capsule, in particular of the type comprising a body attached to the container and a cover cap articulated on the body. It may also be in the form of a member for selectively closing the container, in particular a pump, a valve or a flap valve.

The product may be contained directly in the container, or indirectly. By way of example, the product may be placed on an impregnated support, in particular in the form of a wipe or of a pad, placed (individually or in plurality) in a box or a sachet. Such a support incorporating the product is described, for example, in application WO 01/03538.

The closing member may be coupled to the container by screwing. Alternatively, the coupling between the closing member and the container is done other than by screwing, in particular via a bayonet mechanism, by click-fastening, gripping, welding, bonding or by magnetic attraction. The term "click-fastening" is intended to mean in particular any system involving the crossing of a bead or cord of material by elastic deformation of a portion, in particular of the closing member, followed by return to the elastically unconstrained position of said portion after the crossing of the bead or cord.

The container may be at least partially made of thermoplastic material. By way of examples of thermoplastic materials, mention may be made of polypropylene or polyethylene.

Alternatively, the container is made of non-thermoplastic material, in particular glass or metal (or alloy).

The containing may have rigid walls or deformable walls, in particular in the form of a tube or a tubular bottle.

The container may comprise means intended to bring about or facilitate the distribution of the composition. By way of example, the container may have deformable walls so as to cause the composition to exit in response to a positive pressure inside the container, said positive pressure being caused by elastic (or non-elastic) squeezing of the walls of the container.

The invention will now be described with reference to the following examples given by way of non-limiting illustration. In these examples, unless otherwise indicated, the amounts are expressed as percentages by weight.

### EXAMPLES

### Example 1: Evaluation of the remanence of the

### tensioning effect

The tensioning effect of a dispersion of colloidal silica has already been evaluated in application FR-2 823 113. The ability of the hydroxyalkyl ureas according to the invention to increase the fissuring strength of the tensioning film formed by colloidal silica is evaluated here.

### Protocol

Compositions A to C below were prepared in a manner conventional for those skilled in the art.

| | Composition A | Composition B | Composition C |
|---|---|---|---|
| Hydroxyethyl urea (at 50% in water)⁽¹⁾ | -- | 6% | -- |
| Glycerol | -- | -- | 3% |
| Dispersion of colloidal silica particles⁽²⁾ | 17.1% | 17.1% | 17.1% |
| Surfactants | 3.5% | 3.5% | 3.5% |
| Cyclomethicone | 10% | 10% | 10% |
| Stearyl alcohol | 1% | 1% | 1% |
| Sequestering agent | 0.05% | 0.05% | 0.05% |
| Thickeners | 0.6% | 0.6% | 0.6% |
| Preserving agents | 0.9% | 0.9% | 0.9% |
| Water | qs 100% | qs 100% | qs 100% |

| | | | |
|---|---|---|---|
| ⁽¹⁾Hydrovance^{®} from National Starch ⁽²⁾ Dispersion at 41% by weight in water of colloidal silica particles having a mean particle diameter of 18 nm (Cosmo S-40 from Catalysts & Chemicals) | | | |

The strengthening potential of glycerol and of hydroxyethyl urea was quantified from the measurement of the breaking strength of materials consisting of compositions A to C.

The test consists in applying a compression stress, to breaking, to the material deposited, by means of a film-drawing device, at the surface of a flexible and deformable foam consisting of neoprene 13 mm thick, the amount of material deposited being such that it makes it possible to obtain a film having a thickness of 15 to 30 µm after drying for 24 h. The use of this foam support makes it possible to impose a considerable distortion on the material deposited at the surface, and therefore to quantify its breaking strength. The mechanical compression stress is exerted after drying of the film, by means of a cylindrical punch 1 mm in diameter; the displacement rate of the punch being 0.1 mm/s. The test is carried out using a TA-XT2i texture analyser sold by the company Stable Micro System. A curve of force F (in N) as a function of displacement d (in mm) is thus obtained, from which it is possible to determine the breaking point of the material.

Two parameters are selected in order to quantify the breaking strength of the material:
① F_{break} (N): breaking strength
② W_{break} (J/m²) : breaking energy: corresponds to the surface area measured under the curve F=f(d)/surface area of the punch

### Results

The results obtained are given in the table below:

| Composition tested | W_{break} (J/M²) |
|---|---|
| Composition A | 19 ± 3 |
| Composition B | 109 ± 4 |
| Composition C | 54 ± 2 |

These results demonstrate the strengthening role of the hydroxyalkyl urea used in the composition according to the invention, in the presence of a tensioning agent such as colloidal silica. In addition, the mechanical properties of the film obtained are clearly superior with hydroxyalkyl urea than with a conventional plasticizer such as glycerol.

## Claims

1. Cosmetic composition comprising, in a physiologically acceptable medium suitable for topical application to facial skin:
(a) at least one tensioning agent chosen from:
- colloidal particles of silica or of silica-alumina composite
- synthetic polymer chosen from interpenetrating polymer networks comprising a polyurethane polymer and a polyacrylic polymer, and
(b) at least one compound chosen from:
- hydroxyalkyl ureas corresponding to general formula (I): 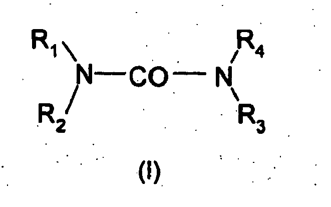 in which R₁, R₂, R₃ and R₄ each independently represent a hydrogen atoms, a C₁-C₄ alkyl group or a C₂-C₆ hydroxyalkyl group that may contain from 1 to 5 hydroxyl groups, at least one of the radicals R₁-R₄ representing a hydroxyalkyl group, and
- the salts, solvates thereof.

2. Composition according to Claim 1, **characterized in that** R₁ is a hydroxyalkyl group and R₂, R₃ and R₄ represent, independently of one another, a hydrogen atom and a C₁-C₄ alkyl group.

3. Composition according to Claim 2, **characterised in that** R₁ is a hydroxyalkyl group and R₂, R₃ and R₄ each represent a hydrogen atom.

4. Composition according to Claim 1, **characterized in that** the compound of formula (I) is chosen from N-(2-hydroxyethyl) urea; N-(2-hydroxypropyl) urea; N-(3-hydroxypropyl) urea; N-(2,3-dihydroxypropyl) urea; N-(2,3,4,5,6-pentahydroxyhexyl) urea ; N-methyl-N-(1,3,4,5,6-pentahydroxy-2-hexyl) urea; N-methyl-N'-(1-hydroxy-2-methyl-2-propyl) urea; N-(1-hydroxy-1-methyl-2-propyl) urea; N-(1,3-dihydroxy-2-propyl) urea; N-(trishydroxymethylmethyl) urea; N-ethyl-N'-(2-hydroxyethyl) urea; N,N-bis-(2-hydroxy-ethyl) urea; N,N'-bi;s-(2-hydroxyethyl) urea; N,N-bis-(2-hydroxypropyl) ureas; N,N'-bis-(2-hydroxypropyl) urea; N,N-bis-(2-hydroxyethyl)-N'-propyl urea; N,N-bis-(2-hydroxypropyl)-N'-(2-hydroxyethyl) urea; N-tert-butyl-N'-(2-hydroxyethyl)-N'-(2-hydroxypropyl) urea; N-(1,3-dihydroxy-2-propyl)-N'-(2-hydroxyethyl) urea; N,N-bis-(2-hydroxyethyl)-N',N'-dimethyl urea; N,N,N',N'-tetrakis-(2-hydroxyethyl) urea; N',N'-bis-(2-hydroxyethyl)-N',N'-bis-(2-hydroxypropyl) urea.

5. Composition according to Claim 3 or 4, **characterised in that** the hydroxyalkyl urea is N-(2-hydroxyethyl) urea.

6. Composition according to any one of Claims 1 to 5, **characterized in that** it contains from 1 to 10% by weight of hydroxyalkyl area, relative to the total weight, of the composition.

7. Composition according to any one of Claims 1 to 6, **characterized in that** said tensioning agent is included in the composition in an amount ranging from 1% to 10% by weight of active material, relative to the total weight of the composition.

8. Process for cosmetically treating wrinkled skin, comprising a step consisting in applying to said skin a composition comprising a hydroxyalkyl urea as defined in any one of Claims 1 to 5 and at least one tensioning agent as defined in Claim 1.

9. Cosmetic assembly comprising:
a) a container delimiting at least one compartment, said container being closed by means of a closing member; and
b) a composition placed inside said compartment, the composition being as defined in any one of Claims 1 to 7.

## Patentansprüche

1. Kosmetische Zusammensetzung, die in einem physiologisch akzeptablen, für eine topische Anwendung auf die Gesichtshaut geeigneten Medium enthält:
(a) mindestens ein Straffungsmittel, das ausgewählt ist unter:
- kolloidalen Partikeln von Siliciumdioxid oder einem Siliciumdioxid/Aluminiumoxid-Verbundmaterial,
- einem synthetischen Polymer, das unter den interpenetrierenden polymeren Netzwerken ausgewählt ist, die ein Polyurethanpolymer und ein Polyacrylpolymer enthalten, und
(b) mindestens eine Verbindung, die ausgewählt ist unter:
- Hydroxyalkylharnstoffen der folgenden allgemeinen Formel (I): 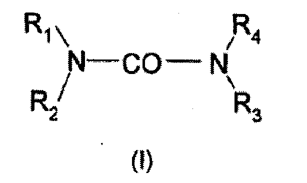 worin R₁, R₂, R₃ und R₄ unabhängig voneinander ein Wasserstoffatom, eine C₁₋₄-Alkylgruppe oder eine C₂₋₆-Hydroxyalkylgruppe bedeuten, die 1 bis 5 Hydroxygruppen enthalten kann, wobei mindestens eine der Gruppen R₁ bis R₄ eine Hydroxyalkylgruppe bedeutet,
- ihren Salzen, Solvaten.

2. Zusammensetzung nach Anspruch 1, **dadurch gekennzeichnet, dass** die Gruppe R₁ eine Hydroxyalkylgruppe ist und die Gruppen R₂, R₃ und R₄ unabhängig voneinander ein Wasserstoffatom und eine C₁₋₄-Alkylgruppe bedeuten.

3. Zusammensetzung nach Anspruch 2, **dadurch gekennzeichnet, dass** die Gruppe R₁ eine Hydroxyalkylgruppe ist und die Gruppen R₂, R₃ und R₄ jeweils ein Wasserstoffatom bedeuten.

4. Zusammensetzung nach Anspruch 1, **dadurch gekennzeichnet, dass** die Verbindung der Formel (I) ausgewählt ist unter:
N-(2-Hydroxyethyl)-harnstoff;
N-(2-Hydroxypropyl)-harnstoff;
N-(3-hydroxypropyl)-harnstoff;
N-(2,3-Dihydroxypropyl)-harnstoff;
N-(2,3,4,5,6-Pentahydroxyhexyl)-harnstoff;
N-Methyl-N-(1,3,4,5,6-pentahydroxy-2-hexyl)-harnstoff;
N-Methyl-N'-(1-hydroxy-2-methyl-2-propyl)-harnstoff;
N-(1-Hydroxy-2-methyl-2-propyl)-harnstoff;
N-(1,3-Dihydroxy-2-propyl)-harnstoff;
N-(tris-Hydroxymethyl-methyl)-harnstoff;
N-Ethyl-N'-(2-hydroxyethyl)-harnstoff;
N,N-Bis(2-hydroxyethyl)-harnstoff;
N,N'-Bis(2-hydroxyethyl)-harnstoff;
N,N-Bis(2-hydroxypropyl)-harnstoff;
N,N'-Bis(2-hydroxypropyl)-harnstoff;
N,N-Bis(2-hydroxyethyl)-N'-propyl-harnstoff;
N,N-Bis(2-hydroxypropyl)-N'-(2-hydroxyethyl)-harnstoff;
N-tert-Butyl-N'-(2-hydroxyethyl)-N'-(2-hydroxypropyl)-harnstoff;
N-(1,3-dihydroxy-2-propyl)-N'-(2-hydroxyethyl)-harnstoff;
N,N-Bis(2-hydroxyethyl) -N',N'-dimethyl-harnstoff;
N,N,N',N'-Tetrakis-(2-hydroxyethyl)-harnstoff;
N',N'-Bis(2-hydroxyethyl)-N',N'-bis-(2-hydroxypropyl)-harnstoff.

5. Zusammensetzung nach Anspruch 3 oder 4, **dadurch gekennzeichnet, dass** der Hydroxyalkylharnstoff der N-(2-Hydroxyethyl)-harnstoff ist.

6. Zusammensetzung nach einem der Ansprüche 1 bis 5, **dadurch gekennzeichnet, dass** sie 1 bis 10 Gew.-% Hydroxyalkylharnstoff, bezogen auf das Gesamtgewicht der Zusammensetzung, enthält.

7. Zusammensetzung nach einem der Ansprüche 1 bis 6, **dadurch gekennzeichnet, dass** das Straffungsmittel in der Zusammensetzung in einer Menge der wirksamen Substanz von 1 bis 10 Gew.-%, bezogen auf das Gesamtgewicht der Zusammensetzung, enthalten ist.

8. Verfahren zur kosmetischen Behandlung faltiger Haut, das einen Schritt umfasst, der darin besteht, eine Zusammensetzung, die einen Hydroxyalkylharnstoff, wie er in einem der Ansprüche 1 bis 5 definiert ist, und mindestens ein Straffungsmittel enthält, wie es in Anspruch 1 definiert ist, auf die Haut aufzutragen.

9. Kosmetische Einheit, enthaltend:
a) einen Behälter, der mindestens eine Abteilung begrenzt, wobei der Behälter durch mindestens ein Verschlusselement verschlossen ist; und
b) eine in der Abteilung befindliche Zusammensetzung, wobei es sich bei der Zusammensetzung um eine Zusammensetzung handelt, wie sie in einem der Ansprüche 1 bis 7 definiert ist.

## Revendications

1. Composition cosmétique comprenant, dans un milieu physiologiquement acceptable adapté à une application topique sur la peau du visage :
(a) au moins un agent tenseur choisi parmi :
- les particules colloïdales de silice ou de composite silice-alumine
- les polymères synthétiques choisis parmi les réseaux de polymères interpénétrés comprenant un polymère polyuréthanne et un polymère polyacrylique, et
(b) au moins un composé choisi parmi :
- les hydroxyalkyl urées répondant à la formule générale (I) : 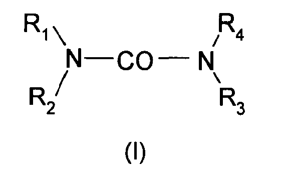 dans laquelle R₁, R₂, R₃ et R₄ représentent chacun indépendamment un atome d'hydrogène, un groupe alkyle en C₁-C₄ ou un groupe hydroxyalkyle en C₂-C₆ pouvant contenir de 1 à 5 groupes hydroxyle, au moins l'un des radicaux R₁-R₄ représentant un groupe hydroxyalkyle, et
- leurs sels, solvats.

2. Composition selon la revendication 1, **caractérisée en ce que** R₁ est un groupe hydroxyalkyle et R₂, R₃ et R₄ représentent, indépendamment les uns des autres, un atome d'hydrogène ou un groupe alkyle en C₁-C₄.

3. Composition selon la revendication 2, **caractérisée en ce que** R₁ est un groupe hydroxyalkyle et R₂, R₃ et R₄ représentent chacun un atome d'hydrogène.

4. Composition selon la revendication 1, **caractérisée en ce que** le composé de formule (I) est choisi parmi la N-(2-hydroxyéthyl)-urée ; la N-(2-hydroxypropyl)-urée ; la N-(3-hydroxypropyl)-urée ; la N-(2,3-dihydroxypropyl)- urée ; la N-(2,3,4,5,6-pentahydroxyhexyl)- urée ; la N-méthyl-N-(1,3,4,5,6-pentahydroxy-2-hexyl)- urée ; la N-méthyl-N'-(1-hydroxy-2-méthyl-2-propyl)- urée ; la N-(1-hydroxy-2-méthyl-2-propyl)-urée ; la N-(1,3-dihydroxy-2-propyl)- urée ; la N-(tris-hydroxyméthyl-méthyl)-urée ; la N-éthyl-N'-(2-hydroxyéthyl)- urée ; la N,N-bis-(2-hydroxyéthyl)- urée ; la N,N'-bis-(2-hydroxyéthyl)- urée ; la N,N-bis-(2-hydroxypropyl)- urée ; la N,N'-Bis-(2-hydroxypropyl)-urée ; la N,N-Bis-(2-hydroxyéthyl)-N'-propyl- urée ; la N,N-Bis-(2-hydroxypropyl)-N'-(2-hydroxyéthyl)- urée ; la N-tert.Butyl-N'-(2-(hydroxyéthyl)-N'-(2-(hydroxypropyl)- urée ; la N-(1,3-dihydroxy-2-propyl)-N'-(2-hydroxyéthyl)- urée; la N,N-Bis-(2-hydroxyéthyl)-N,N'-dimethyl- urée ; la N,N,N',N'-tetrakis-(2-hydroxyéthyl)- urée ; la N',N'-Bis-(2-hydroxyéthyl)-N',N'-bis-(2-hydroxypropyl)- urée.

5. Composition selon la revendication 3 ou 4, **caractérisée en ce que** l'hydroxyalkyl urée est la N-(2-hydroxyéthyl)-urée.

6. Composition selon l'une quelconque des revendications 1 à 5, **caractérisée en ce qu'**elle renferme de 1 à 10% en poids d'hydroxyalkyl urée, par rapport au poids total de la composition.

7. Composition selon l'une quelconque des revendications 1 à 6, **caractérisée en ce que** ledit agent tenseur est compris dans la composition en une teneur allant de 1% à 10% en poids de matière active, par rapport au poids total de la composition.

8. Procédé de traitement cosmétique d'une peau ridée comprenant une étape consistant à appliquer sur ladite peau une composition comprenant une hydroxyalkyl urée telle que définie dans l'une quelconque des revendications 1 à 5 et au moins un agent tenseur tel que défini dans la revendication 1.

9. Ensemble cosmétique comprenant :
a) un récipient délimitant au moins un compartiment, ledit récipient étant fermé par un élément de fermeture ; et
b) une composition disposée à l'intérieur dudit compartiment, la composition étant conforme à l'une quelconque des revendications 1 à 7.
